# EUROPEAN PATENT SPECIFICATION

(11) **EP 1 743 583 B1**
(45) Date of publication and mention of the grant of the patent: **04.03.2009**
(21) Application number: 06013777.5
(22) Date of filing: 04.07.2006
(51) Int. Cl.: A61B 17/34

(54) **Surgical instrument for making backward subcutaneous tunnelling**
Chirurgische Vorrichtung zum Rückwärtsmachen von einem subkutanen Tunnel
Instrument chirurgical pour ouvrir par retour un tunnel sous cutané

(30) Priority: 14.07.2005 IT MI20050256
(43) Date of publication of application: 17.01.2007
(73) Proprietor: BIOENGINEERING LABORATORIES S.p.A., 22063 Cantu' CO (IT)
(72) Inventor: Galli, Franco c/o BEL S.p.A., I-22063 Cantu (CO) (IT); Greco, Francesco, I-22063 Cantu (CO) (IT)
(74) Representative: Ferraiolo, Ruggero

(56) References cited:
- WO-A-98/35616
- GB-A- 2 103 936
- GB-A- 2 214 814
- US-A- 4 826 477

## Description

The present patent application refers to a surgical instrument for making a backward subcutaneous tunnelling subsequent to the introduction of a central venous catheter.

The technical field of the present application is that of surgical instruments which let a central venous catheter be drawn from the point at which the catheter emerges from a vein to the point at which the catheter emerges from the skin, making the catheter to follow a subcutaneous path.

The known instruments are made of two rectilinear bars where a first bar has a pointed end provided with roughness's that make a catheter end to be retained by said bar end and a second bar has an olive-shaped end adapted to receive the pointed end of the first bar. The use of such instruments is well known to those who operate in the field and will be described, in a brief way only, with reference to the invented instrument. The known instruments are adapted to set the catheter in a substantially rectilinear position; it's just said feature that makes unstable the position given the catheter during the operation since the rectilinear seat let the catheter slide easily therein.

Document WO 98/35616 A is known that relates to devices and methods for use in percutaneous and hiatal approaches treatments for urinary incontinence wherein there are comprised guide members placement devices, sling application catheters, tissue dissectors/dilators, sling application devices and a sling application system, tissue expanders, grasping devices and balloon catheters. It may be relevant to remark that two hollow bars are provided which are each inserted in opposite positions in a human body area and couple therein in rectilinear relation for receiving a guide member adapted to draw a sling, The instrument according to this application obviates the above mentioned drawback and solves the problem to place a catheter in a seat adapted to prevent the catheter from sliding, once it is installed.

The instrument conventionally comprises two elements that are called conventionally, the first, "catheter tunnelling element" or more simply "tunnelling element", the second "counter-tunneling element for the achoring swell" or more simply "counter-tunneling element".

It is known that catheters are used that are provided with an anchoring swell, namely with a sort of sleeve, generally made in "Dacron" ® along the length of the outer catheter wall and generally about the middle of the length, with the purpose of working as an anchorage for the catheter and preventing it from sliding. In fact, once the surgeon works for setting the catheter in the operating field, he makes the sleeve causing a sort of recess wherein the catheter should be kept still, thanks to the elasticity of the tissue wherein it is received. In spite of that, the known instruments are subject to sliding along the seat.

The instrument according to this application obviates the said drawback.

The tunnelling element is a curved metal bar of a diameter equal with the catheter outer diameter; one of its ends is pointed and is provided with roughness's. The catheter end rising from the skin may engage on said pointed end so that the catheter may be drawn by the tunnelling instrument as a surgeon makes the latter slide along its curved seat.

The counter-tunnelling element also is a curved metal bar having a hollow olive-shaped end of an outer diameter equal with the diameter of the catheter anchorage sleeve; the olive hollow serves to receive the pointed end of the tunnelling element so making continuity between the two bars.

The curved shapes of said instrument make feasible subcutaneous tunnels of curved progression in order to strengthen the catheter anchorage in its seat, due to the increased friction, in the position given by the surgeon. This advantage is notable in the concerned pulling condition.

The anchoring system is usually a sleeve of a diameter larger that the catheter diameter, the sleeve being drawn under the skin together with the catheter; due to its large diameter the sleeve would meet resistances against moving forward when drawn together with the tunnelling element only. The use of the counter-tunnelling element helps such moving as it causes room enough for the sleeve setting, before tunnelling the whole catheter.

The instruments used as follows.

A tunnellig element is introduced in the point where a catheter which was previously inserted in a central vein of a body rises from the skin; attention shall be paid to the fact that the pointed end of the tunnelling element gets on the rising point of the catheter. The curved pointed end of the counter-tunelling element is forced on the tip of the tunnelling element, the counter-tunelling element being pushed in the contrary direction and driven by the tunnelling element up to the level selected for setting the catheter sleeve in the desired position. Moving again in the contrary direction, the tunnelling element moves forward upto the point where it rises from the catheter, it is made free from the counter-tunnelling, it hooks the catheter and draws it along the subcutaneous path, just made. In such a way the sleeve, being of a diameter higher than that of the tunnelling element, reaches the selected seat once the catheter is drawn and is not allowed to further move since it finds a canal of reduced dimensions.

The invention will be described now in detail by means of the accompanying drawings where
- Fig. 1 is a side view of a tunnelling element,
- Fig. 2 is a side view of a couter-tunnelling element,
- Fig. 3 is a side view of tunnelling and couter-tunnelling elements, joined together.

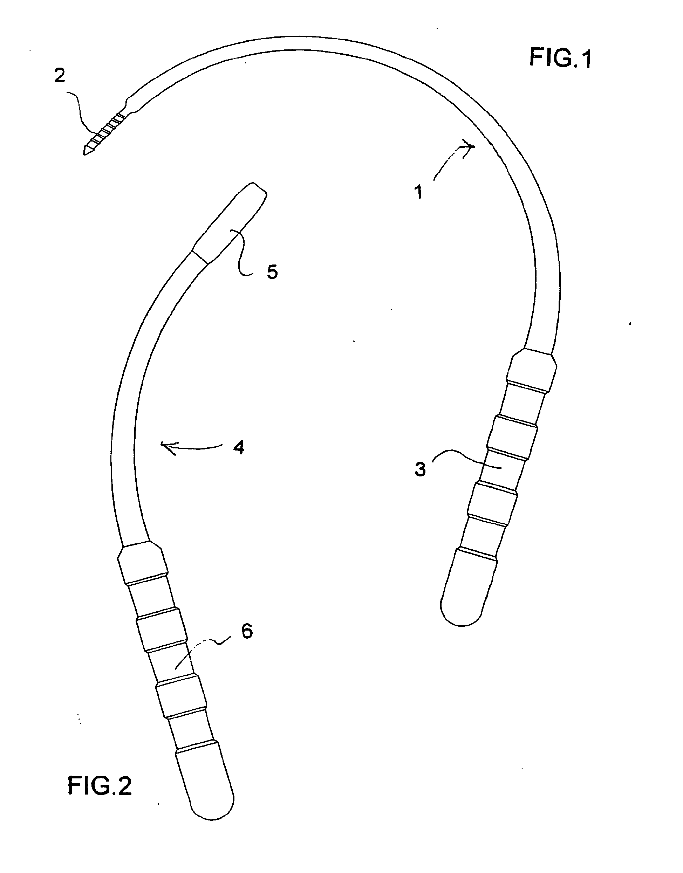

Fig. 1 shows a tunnelling element 1 having a desired curvature; the left end 2 is a pointed one and has roughness's as a rough threading , whilst the right end bears a handle 3.

Fig. 2 shows a counter-tunnelling element 4 having a desired curvature, substantially equal with the curvature of the tunnelling element; the upper end is shaped as a hollow olive 5 of outer diameter equal with the diameter of the catheter anchoring sleeve, not shown in the figure; the olive hollow is adapted to receive the tunnelling element tip so as to make a continuity between the two bars.

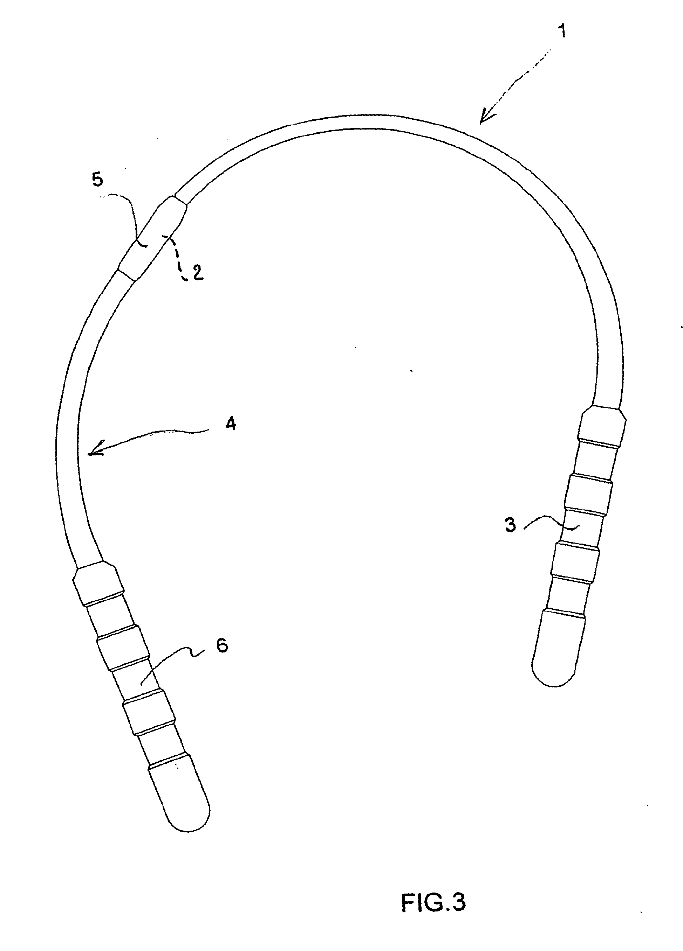

Fig. 3 shows the two elements 1 and 4 joined by the engagement of tip 2 into the hollow olive 5, as it occurs in one of the steps for using the instrument, as described previously.

It is realized that an operator in the concerned medical field will be equipped with a set of instruments of different curvatures which anables him to operate in any specific operation area.

## Claims

1. Surgical system comprising a catheter, a catheter anchoring sleeve and a surgical tunnelling instrument for making a backward subcutaneous tunnelling subsequent to the introduction of the central venous catheter, the surgical tunnelling instrument comprising a tunnelling element (1) and a counter-tunneling element (4), wherein
the tunnelling element (1) is provided with a shaft whose outer diameter is equal to the catheter outer diameter and a pointed end (2) having roughness's that make the catheter end to be retained by said tunnelling element end (2),
the counter tunnelling element (4) has a shaft and an olive-shaped end (5) adapted to receive the pointed end (2) of the tunnelling element (1) and whose outer diameter is equal to the diameter of the catheter anchoring sleeve, and
the tunnelling element (1) and the counter -tunnelling element (4) have a desired curvature.

2. Surgical system according to claim 1 wherein the curvatures of said elements (1, 4) are different one from another.

## Patentansprüche

1. Chirurgisches System mit einem Katheter, einer Katheterverankerungshülse und einem chirurgischen Tunnelbildungsinstrument zur Durchführung einer rückwärtsgerichteten subkutanen Tunnelbildung nach dem Einführen des zentralen Venenkatheters, wobei das chirurgische Tunnelbildungsinstrument ein Tunnelelement (1) und ein Gegentunnelelement (4) aufweist, wobei
das Tunnelelement (1) mit einem Schaft, dessen Außendurchmesser gleich dem Katheteraußendurchmesser ist, und einem zugespitzten Ende (2) versehen ist, das eine Rauhigkeit aufweist, die bewirkt, dass das Katheterende durch das Tunnelelementende (2) zurückgehalten wird,
das Gegentunnelelement (4) einen Schaft und ein olivenförmiges Ende (5) hat, das so ausgebildet ist, dass es das zugespitzte Ende (2) des Tunnelelementes (1) aufnehmen kann, und dessen Außendurchmesser gleich dem Durchmesser der Katheterverankerungshülse ist, und
das Tunnelelement (1) und das Gegentunnelelement (4) eine gewünschte Krümmung haben.

2. Chirurgisches System nach Anspruch 1, wobei die Krümmungen der Elemente (1, 4) sich voneinander unterscheiden.

## Revendications

1. Système chirurgical comprenant un cathéter avec son manchon de fixation et un instrument chirurgical pour ouvrir par retour un tunnel sous-cutané suite à l'introduction d'un cathéter veineux central, l'instrument chirurgical d'ouverture d'un tunnel comprenant un élément d'ouverture d'un tunnel (1) et un élément antagoniste (4) d'ouverture d'un tunnel,
l'élément d'ouverture d'un tunnel (1) est muni d'une tige dont le diamètre extérieur est égal au diamètre extérieur du cathéter et d'une extrémité (2) en forme de pointe dont la rugosité permet à l'extrémité du cathéter d'être retenue par l'extrémité (2) de l'élément d'ouverture d'un tunnel,
l'élément antagoniste (4) d'ouverture d'un tunnel est munie d'une tige et d'une extrémité en forme d'olive (5) apte à recevoir l'extrémité en forme de pointe (2) de l'élément d'ouverture d'un tunnel (1) et dont le diamètre extérieur est égal au diamètre du manchon de fixation du cathéter, et
l'élément d'ouverture d'un tunnel (1) et l'élément antagoniste (4) d'ouverture d'un tunnel ont la courbure désirée.

2. Système chirurgical selon la revendication 1,
dans lequel
les courbures des éléments (1, 4) sont différentes l'une de l'autre.
